# EUROPÄISCHE PATENTANMELDUNG

(11) **EP 0 943 292 A1**
(43) Veröffentlichungstag der Anmeldung: **22.09.1999**
(21) Anmeldenummer: 99105276.2
(22) Anmeldetag: 15.03.1999
(51) Int. Cl.: A61B 10/00

(54) **Spiralsonde**

(30) Priorität: 19.03.1998 DE 19812100
(71) Anmelder: B. BRAUN MELSUNGEN AG, 34212 Melsungen (DE)
(72) Erfinder: Frimberger, Eckart, Dr., 80804 München (DE)
(74) Vertreter: Körber, Wolfhart, Dr.

(57) **Zusammenfassung**

Eine Sonde zur Entnahme von Körperzellen und/oder Körperflüssigkeiten aus Hohlorganen weist eine Hohlsonde (1) und eine mit der Hohlsonde (1) verbundene Hohlspirale (2) auf, deren Spiralwicklungen einen solchen Abstand voneinander aufweisen, daß Gewebematerial und/oder Körperflüssigkeit in den im Zentrum der Hohlspirale (2) gebildeten Hohlraum (12) eindringen kann. Die Sonde ermöglicht eine effiziente und schnelle Gewinnung von Gewebematerial und/oder Körperflüssigkeit. Die Effizienz kann durch Erzeugung eines Vakuums in der Sonde (1, 2) noch erhöht werden.

## Beschreibung

Die Erfindung betrifft eine Sonde zur Entnahme Von Körperzellen und/oder Körperflüssigkeiten aus Hohlorganen mit einer Trägersonde, eine mit der Trägersonde verbundene Hohlspirale aus einem Flachbandmaterial, deren Spiralwindungen einen solchen Abstand voneinander aufweisen, daß Gewebematerial und/oder Körperflüssigkeit in den im Zentrum der Hohlspirale gebildeten Hohlraum eindringen kann.

Zur Entnahme von Körperzellen aus Hohlorganen zur anschließenden Untersuchung, beispielsweise für die Tumordiagnostik, ist es bekannt, sogenannte "Zytologie-Bürsten" zu verwenden. Dabei handelt es sich um feine, am Ende einer Sonde angebrachte Bürsten, die beispielsweise mit einem Endoskop an die gewünschte Stelle im Körper eingeführt werden. Die Bürste an der Spitze der Sonde weist einen zentralen Drahtabschnitt auf, an dem in regelmäßigen Abständen radial nach außen abstehende Bürstenhaare aus Kunststoff oder dergleichen angeordnet sind. Durch Hin- und Herbewegen der Bürste in dem betreffenden Bereich des Hohlorgans, beispielsweise im vermuteten Tumorgebiet, werden Zellen abgerieben, die an den Bürstenhaaren haften bleiben und nach Zurückziehen der Sonde beispielsweise durch Eintauchen in eine konservierende Flüssigkeit entfernt und anschließend analysiert werden können. Da nur ein kleiner Teil des abgeriebenen Zellmaterials an den Bürstenhaaren haftet und von diesem dann auch beim Ablösevorgang noch ein Teil verlorengeht, läßt sich mit dieser Methode nur wenig Material gewinnen, wodurch deren Effizienz erheblich eingeschränkt ist.

Aus der US-A 3,683,891 ist eine Trägersonde aus Draht und eine mit der Trägersonde verbundene Hohlspirale aus einem Flachbandmaterial bekannt. Die Spiralwindungen weisen einen solchen Abstand auf, daß Gewebematerial in den im Zentrum der Hohlspirale gebildeten Hohlraum eindringen können. Eine Entnahme von Gewebematerial mit dieser Sonde ist nur dann möglich, wenn das Gewebematerial an den Spiralwindungen hängen bleibt.

Zu Analysezwecken werden häufig Flüssigkeiten oder Sekrete aus Hohlorganen entnommen. Es ist bekannt, diese Flüssigkeiten mit Schlauchsonden abzusaugen, die an ihrem Endbereich seitliche Perforationen aufweisen, durch die mittels eines in der Sonde erzeugten Vakuums die Flüssigkeit abgesaugt wird. Bei diesem Verfahren tritt jedoch das Problem auf, daß sich beim Absaugen sehr leicht Schleimhaut von der Innenwand des Hohlorgans an die Perforationen anlegt und diese okkludiert. Dies hat zur Folge, daß die Flüssigkeit nicht vollständig abgesaugt werden kann und daher eine quantitative Analyse, beispielsweise des Sekrets der Bauchspeicheldrüse, nicht möglich ist.

Der vorliegenden Erfindung liegt daher die Aufgabe zugrunde, eine Sonde vorzuschlagen, die eine erhöhte Effizienz und ein gewebeschonendes Einführen und Entnahme von Körperzellen und/oder Körperflüssigkeiten aus Hohlorganen ermöglicht.

Gelöst wird diese Aufgabe durch eine Sonde mit den Merkmalen des unabhängigen Anspruches 1 oder des unabhängigen Anspruches 2. Weitere Ausgestaltungen der Erfindung sind in den jeweils rückbezogenen Unteransprüchen gekennzeichnet.

Die von der Spirale "abgehobelten" Gewebepartikel dringen in den Hohlraum im Innern der Hohlspirale ein und gelangen dann zu der vorzugsweise als Hohlsonde ausgebildeten Trägersonde. Es ist nicht erforderlich, daß die Zellen wie bei den bekannten Sonden an der Hohlspirale hängenbleiben müssen. Dadurch ist die Ausbeute der erfindungsgemäßen Sonde wesentlich erhöht. Zur weiteren Steigerung der Effizienz ist es möglich, ein Vakuum im Innern der Sonde zu erzeugen, wodurch die abgeriebenen Zellen und Körperflüssigkeit abgesaugt werden können.

Die umlaufende Öffnung der Spiralsonde wird in wesentlich geringerem Maße durch die Schleimhaut des zu untersuchenden Hohlorgans okkludiert als dies bei den kreisförmigen oder ovalen Perforationen einer bekannten Schlauchsonde zur Drainage von Körperflüssigkeit der Fall ist. Dadurch weist die erfindungsgemäße Sonde auch zur Entnahme von Flüssigkeit eine deutlich erhöhte Effizienz auf. Die quantitative Analyse von Körpersekreten wird dadurch erleichtert.

Die Hohlspirale weist eine abgerundete Spitze auf, die ein möglichst atraumatisches Einführen der Sonde erleichtert. Die Spiralsonde kann auch mit einem innen verlaufenden Zugseil versehen sein, das an der Spitze der Spirale befestigt ist. Durch das Zugseil läßt sich die Hohlspirale "zusammenziehen", wodurch sich die Abstände benachbarter Spiralwindungen verringern und die Spirale so geschlossen werden kann. So kann verhindert werden, daß gewonnenes Zellmaterial beim Herausziehen der Sonde aus dem Innenraum der Hohlspirale verlorengeht.

Die Hohlspirale kann abnehmbar an der Trägersonde befestigt sein, welche vorzugsweise als Kunststoffschlauch ausgebildet ist.

Die Trägersonde kann an eine Vakuumpumpe anschließbar sein. Dadurch wird die Gewinnung von Gewebematerial und insbesondere von Körperflüssigkeiten verbessert.

Die Sonde kann eine im Innern der Trägersonde und der Hohlspirale angeordnete Innenhohlsonde aufweisen, die vorzugsweise im vorderen Bereich der Hohlspirale endet und gegenüber der Trägersonde abgedichtet ist. Mit der Innenhohlsonde ist es möglich, einen definierten Flüssigkeitsstrom entlang der an der Spiralsonde anhaftenden Organinnenwand aufrechtzuerhalten und einen Stoffaustausch an der Innenwand des Hohlorgans quantitativ zu untersuchen.

Die Erfindung wird im folgenden anhand bevorzugter Ausführungsbeispiele unter Bezugnahme auf die Zeichnung im Detail erläutert, in der
- Figur 1: ein erstes Ausführungsbeispiel der erfindungsgemäßen Sonde zeigt;
- Figur 2: ein zweites Ausführungsbeispiel der erfindungsgemäßen Sonde zeigt;
- Figur 3: ein drittes Ausführungsbeispiel der erfindungsgemäßen Sonde zeigt;
- Figur 4: ein viertes Ausführungsbeispiel der erfindungsgemäßen Sonde zeigt;
- Figur 5: ein fünftes Ausführungsbeispiel der erfindungsgemäßen Sonde zeigt.

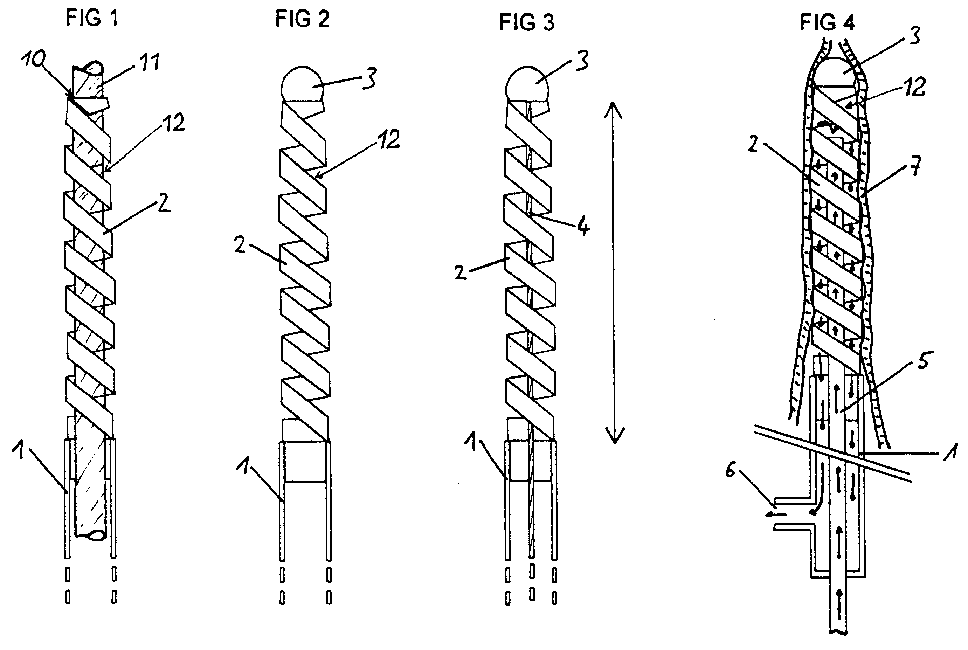

Figur 1 zeigt ein bevorzugtes Ausführungsbeispiel der erfindungsgemäßen Sonde zur Entnahme von Körperzellen und/oder Körperflüssigkeiten aus Hohlorganen. Die Sonde besteht aus einer als Hohlsonde 1 ausgebildeten Trägersonde, an deren Spitze eine Hohlspirale 2, die einen Hohlraum 12 definiert, befestigt ist. Die Hohlspirale 2 kann zum Zwecke des Austauschs durch eine andere Spirale entfernbar an der Hohlsonde 1 angebracht sein, es ist jedoch darauf zu achten, daß die Verbindung sehr fest ist, so daß ein versehentliches Lösen der Hohlspirale 2 während der Untersuchung ausgeschlossen ist. Die Trägersonde ist wie gesagt als Hohlsonde 1, beispielsweise als transparenter Plastikschlauch, ausgebildet. Die Hohlspirale 2 ist beispielsweise aus elastischem Metall oder Kunststoffmaterial ausgebildet, wobei die Spiralwindungen einen solchen Abstand voneinander aufweisen, daß Gewebematerial und Körperflüssigkeit in den im Zentrum der Hohlspirale 2 gebildeten Hohlraum 12 eindringen kann. Die Hohlspirale 2 kann einen insgesamt zylindrischen oder auch einen sich zum vorderen und/oder hinteren Ende konisch verjüngenden Querschnitt aufweisen. Die in Figur 1 gezeigte Sonde weist am vorderen Ende der Hohlspirale 2 eine Öffnung auf, so daß die Sonde über einen Führungsdraht 11 ins Zielgebiet geschoben werden kann. Der Führungsdraht 11 füllt die Öffnung 10 aus und ist an seinem vorderen nicht dargestellten Ende so ausgebildet, daß die Hohlspirale 2 atraumatisch an das Gewebe des Hohlorgans herangeführt werden kann, entweder zusammen mit dem Führungsdraht 11 oder durch Verschieben auf dem Führungsdraht.

Die in Figur 2 gezeigte erfindungsgemäße Sonde weist eine abgerundete Spitze 3 auf, die den Hohlraum 12 der Hohlspirale 2 verschließt und ein möglichst atraumatisches Einführen der Sonde in das gewünschte Zielgebiet erleichtert.

Figur 3 zeigt eine Ausführungsform der erfindungsgemäßen Entnahmesonde, die wie bei dem in Figur 1 gezeigten Ausführungsbeispiel eine abgerundete Spitze 3 hat. An dieser ist jedoch ein Zugseil 4 befestigt. Durch Zug an diesem Zugseil 4 kann die Spirale 2 zusammengezogen werden, wodurch sich die Abstände zwischen den einzelnen Spiralwindungen verringern oder ganz schließen. Die Sonde kann dann in geschlossenen Zustand zurückgezogen werden, wobei ein Verlieren von gewonnenen Gewebepartikeln vermieden wird. Um diese Öffen- und Schließfunktion der Spirale mittels des Zugseils 4 zu realisieren sind Material und Ausmaße der Spiralwindungen vorzugsweise so gewählt, daß eine mäßige Zugkraft am Zugseil 4 ausreicht, um die Spirale 2 zusammenzuziehen.

Figur 4 zeigt ein Ausführungsbeispiel der erfindungsgemäßen Entnahmesonde, bei dem die Sonde eine zusätzliche Innenhohlsonde 5 mit kleinerem Durchmesser aufweist, die im Zentrum der Hohlsonde 1 und der Hohlspirale 2 angeordnet ist und vorzugsweise im vorderen Bereich des Hohlraumes 12 endet. Der Innenraum der Innenhohlsonde 5 ist gegenüber dem Innenraum der Hohlsonde 1 abgedichtet. So kann an einem Anschlußstutzen 6 der Hohlsonde 1 außerhalb des Körpers eine Vakuumpumpe angeschlossen werden und so ein Vakuum im Innern der Sonde 1, 2 erzeugt werden. Gleichzeitig ist es möglich, die gegenüber der Hohlsonde 1 abgedichtete Innerhohlsonde 5 an eine Flüssigkeitszuführung anzuschließen. So kann ein gleichmäßiger Flüssigkeitsstrom aus der Innenhohlsonde durch die Hohlspirale entlang der Innenwände 7 des Hohlorgans und weiter durch die Hohlsonde 1 zu dem Vakuumanschluß ermöglicht werden. Ein Flüssigkeitsstrom in entgegengesetzter Richtung ist jedoch ebenfalls möglich.

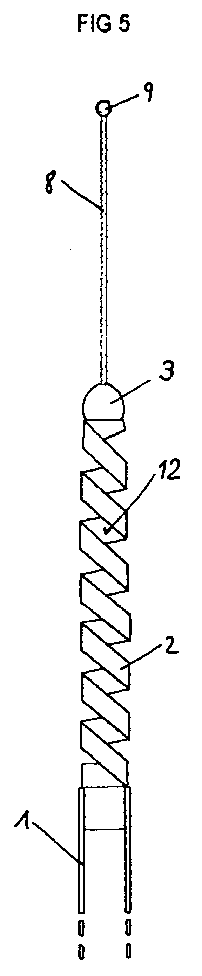

Figur 5 zeigt ein weiteres Ausführungsbeispiel der erfindungsgemäßen Sonde. Am vorderen, distalen Ende der Sonde ist eine sehr flexible Sondenspitze 8, 9 angebracht, die beispielsweise aus einem dünnen Stahlseil mit einem Durchmesser von 0,2 bis 1,2 mm oder einem Nitinoldraht besteht, wobei das vorderste Ende durch eine Kugel 9 mit einem Durchmesser von beispielsweise 0,5 mm bis 1,5 mm gebildet werden kam. Die sehr flexible Spitze erleichtert die Einführung der Sonde in stark verwinkelte Engstellen des Körpers.

Im folgenden werden Anwendungsmöglichkeiten der erfindungsgemäßen Entnahmesonde kurz erläutert.

Bei der Anwendung zur Zytologie wird die erfindungsgemäße Sonde beispielsweise durch den Instrumentierkanal eines Endoskops in das Zielgebiet vorgeschoben und die Spirale im Zielgebiet bin- und herbewegt, wodurch Zellen von dem zu untersuchenden Hohlorgan abgelöst werden und in das Innere der Hohlspirale 2 und der Hohlsonde 1 gelangen. Daraufhin wird die Sonde wieder zurückgezogen und das in den Innenraum der Hohlspirale 2 eingetretene Zellmaterial kann durch Schütteln in ein Gefäß mit Flüssigkeit oder durch Spülen der Hohlspirale 2 von ihrem hinteren Ende her gewonnen werden. Im Vergleich mit der bekannten Bürstenzytologie kann durch die erfindungsgemäße Sonde die mehrfache Menge an Zellmaterial gewonnen werden. Eine weitere Möglichkeit der Steigerung der Ausbeute besteht darin, während des Entnahmevorgangs ein Vakuum im Innenraum der Sonde zu erzeugen, wodurch das Gewebe an die Hohlspirale 2 an- und teilweise in die Spirale 2 eingesogen wird. Da die bekannte Bürstensonde keinen Innenhohlraum aufweist, ist dort die Erzeugung eines Soges zur Steigerung der Ausbeute nicht möglich.

Bei der Anwendung zur Gewinnung von Flüssigkeit wird die erfindungsgemäße Sonde auf ähnliche Weise, beispielsweise mittels eines Endoskops, an das gewünschte Zielgebiet eingeführt. Die zirkuläre Durchtrittsöffnung der Hohlspirale 2 verhindert eine Okklusion und ermöglicht so die vollständige Gewinnung der Flüssigkeit. Diese kann über die als Hohlsonde ausgebildete Trägersonde abgesaugt werden. Ein Spezialfall dieser Anwendung liegt in der Versorgung von sogenannten "Anastomosen-Lecks" . Wird bei einer Operation ein Hohlorgan durchtrennt und anschließend wieder verbunden, beispielsweise nach Entfernung eines Darmstückes, kann es an der Verbindungsstelle, der Anastomose, zu einer Undichtigkeit bzw. einem Leck kommen. Durch diese undichte Verbindungsstelle tritt Flüssigkeit in die Umgebung aus, was eine schwerwiegende Komplikation darstellen kann. Die erste therapeutische Maßnahme besteht dann in der Plazierung von Absaugsonden in dem betroffenen Hohlorgan. Auch hier legt sich die Schleimhaut beim Absaugen an die Öffnungen der Sonde an, was ein vollständiges Absaugen verhindert. Durch die erfindungsgemäße Sonde ist somit auch in diesem Fall eine effizientere Absaugung und damit eine bessere Versorgung des Patienten möglich.

Ein weiter verbessertes Verfahren zur Gewinnung von Körperzellen aus Hohlorganen ist mit dem in Figur 3 dargestellten vierten Ausführungsbeispiel der erfindungsgemäßen Sonde möglich. Am hinteren Ende der Hohlsonde 1 wird am Stutzen 6 ein Unterdruck angelegt, so daß das Gewebe 7 des Hohlorgans an die Hohlspirale 2 angesogen wird. Durch Hin- und Herbewegen abgescherte Zellen von der Innenwand 7 des Hohlorgans können wahrend des Eingriffs aus dem Körper entfernt werden, wenn über die Innenhohlsonde 5, wie in Figur 4 durch Pfeile angedeutet ist, Flüssigkeit zugeleitet wird. Die Absaugleistung an der Hohlsonde 1 muß dann ausreichend groß sein, damit trotz Einleitung der Flüssigkeit über die Innenhohlsonde 5 ein Sog aufrechterhalten bleibt. Die durch Abscheren gewonnen Gewebepartikel werden dann mit der Flüssigkeit abgesaugt und können außerhalb des Körpers gewonnen und analysiert werden.

Eine weitere Anwendungsmöglichkeit der erfindungsgemäßen Sonde mit Innenhohlsonde 5 ist ein neuartiges Verfahren zur Analyse des Stoffaustausches an der Innenwand von Hohlorganen. An dem Ansaugstutzen 6 der Trägersonde 1 wird wiederum ein Vakuum angelegt, so daß sich die Schleimhaut 7 des betreffenden Hohlorgans an die Hohlspirale 2 anlegt. Die zirkuläre Öffnung der Spirale 2 und die an dieser anliegende Schleimhaut bilden eine definierte Oberfläche in einem abgeschlossenen Hohlraum. Wird in diesem unter Dauersog stehenden Hohlraum über die Innenhohlsonde 5 Flüssigkeit eingeleitet, wobei wiederum die Saugleistung so gewählt ist, daß der Sog in der Hohlspirale 2 aufrechterhalten bleibt, tritt die Flüssigkeit mit der an der Spirale 2 anliegenden Schleimhaut 7 im Bereich der zirkulären Öffnung der Spirale 2 in Kontakt. Sekretions- und Absorptionsvorgänge zwischen der zugeleiteten Flüssigkeit und der Schleimhaut 7 des Organs lassen sich über eine quantitative Analyse der abgesaugten Flüssigkeit ermitteln. Auf diese Weise kann auch die kontinuierlich durch das System perfundierte Flüssigkeit analysiert werden.

Mit der erfindungsgemäßen Sonde wird die Entnahme von Körperzellen und/oder Körperflüssigkeiten aus Hohlorganen verbessert und dessen Ausbeute erhöht. Desweiteren wird ein Verfahren zur quantitativen Analyse des Stoffaustausches an der Innenwand von Hohlorganen vorgeschlagen.

## Patentansprüche

1. Sonde zur Entnahme Von Körperzellen und/oder Körperflüssigkeiten aus Hohlorganen, aufweisend
eine Trägersonde (1),
eine mit der Trägersonde (1) verbundene Hohlspirale (2) aus einem Flachbandmaterial, deren Spiralwindungen einen solchen Abstand voneinander aufweisen, daß Gewebematerial und/oder Körperflüssigkeit in den im Zentrum der Hohlspirale (2) gebildeten Hohlraum (12) eindringen kann,
**dadurch gekennzeichnet,**
daß die Trägersonde als Hohlsonde (1) ausgebildet ist, deren mindestens eine Kanal mit dem Hohlraum (12) der Hohlspirale (2) in Verbindung steht, und
daß die Hohlspirale (2) an ihrem vorderen Ende eine Öffnung (10) zur Aufnahme eines Führungsdrahtes (11) aufweist.

2. Sonde zur Entnahme von Körperzellen und/oder Körperflüssigkeiten aus Hohlorganen, aufweisend
eine Trägersonde (1),
eine mit der Trägersonde (1) verbundene Hohlspirale (2) aus einem Flachbandmaterial, deren Spiralwindungen einen solchen Abstand voneinander aufweisen, daß Gewebematerial und/oder Körperflüssigkeit in den im Zentrum der Hohlspirale (2) gebildeten Hohlraum (12) eindringen kann,
**dadurch gekennzeichnet,**
daß die Trägersonde als Hohlsonde (1) ausgebildet ist, deren mindestens eine Kanal mit dem Hohlraum (12) der Hohlspirale (2) in Verbindung steht, und
daß die Hohlspirale (2) an ihrem vorderen Ende eine ihrem Hohlraum (12) zuschließende, ein atraumatisches Einführen der Sonde ermöglichende abgerundete Spitze (3) aufweist.

3. Sonde nach Anspruch 2, **dadurch gekennzeichnet,** daß die Abstände benachbarter Spiralwindungen der Hohlspirale (2) mittels eines im Zentrum der Hohlsonde (1) vorgesehenen Zugseils (4) veränderbar sind, das an der abgerundeten Spitze (3) befestigt ist.

4. Sonde nach einem der Ansprüche 2 oder 3, **dadurch gekennzeichnet**, daß die Hohlspirale (2) eine flexible Sondenspitze (8, 9) als elastischen Einführungsdraht für die Hohlsonde (1) aufweist.

5. Sonde nach einem der Ansprüche 1 bis 4, **dadurch gekennzeichnet,** daß die Hohlspirale (2) abnehmbar an der Hohlsonde (1) befestigt ist.

6. Sonde nach einem der vorhergehenden Ansprüche, **dadurch gekennzeichnet,** daß die Hohlsonde (1) als Kunststoffschlauch ausgebildet ist.

7. Sonde nach einem der Ansprüche 1 bis 6, **gekennzeichnet durch eine** im Innern der Hohlsonde (1) und der Hohlspirale (2) angeordnete Innenhohlsonde (5), die im vorderen Bereich des Hohlraumes (12) der Hohlspirale (2) endet.

8. Sonde nach Anspruch 7, **dadurch gekennzeichnet,** daß der Innenraum der Innenhohlsonde (5) gegenüber dem Innenraum der Hohlsonde (1) abgedichtet ist.

9. Sonde nach Anspruch 8, **dadurch gekennzeichnet**, daß eines, die Hohlsonde (1) oder die Innenhohlsonde (5) mit einer Vakuumpumpe und das andere die Innerhohlsonde (5) oder die Hohlsonde (1) mit einer Flüssigkeitszuführung verbunden ist, wobei die Absaugleistung der Vakuumpumpe so einstellbar ist, das trotz Flüssigkeitszuführung in dem Hohlraum (12) der Hohlspirale (2) ein Unterdruck aufrechterhalten wird.

10. Sonde nach Anspruch 9, **gekennzeichnet durch** die Zuführung einer solchen definierten Flüssigkeitsmenge an den Hohlraum (12) der Hohlspirale (2), daß trotz Flüssigkeitszuführung die Innenwände (7) des die Hohlspirale (2) aufnehmenden Hohlorgans in Kontakt mit der Hohlspirale (2) bleiben.
